# EUROPÄISCHE PATENTSCHRIFT

(11) **EP 3 655 050 B1**
(45) Veröffentlichungstag und Bekanntmachung des Hinweises auf die Patenterteilung: **17.01.2024**
(21) Anmeldenummer: 18745555.5
(22) Anmeldetag: 20.07.2018
(51) Int. Cl.: A61L 15/18, A61L 15/42, A61L 15/44

(54) **NEUARTIGE WUNDAUFLAGE ZUR BLUTSTILLUNG**
NEW KIND OF WOUND DRESSING FOR HAEMOSTASIS
NOUVEAU PANSEMENT POUR L'HÉMOSTASE

(30) Priorität: 21.07.2017 DE 102017116511
(43) Veröffentlichungstag der Anmeldung: 27.05.2020
(73) Patentinhaber: Speed Care Mineral GmbH, 17034 Neubrandenburg (DE)
(72) Erfinder: SCHOMBURG, Joachim, 17034 Neubrandenburg (DE); LÖFFLER, Marita, 17087 Altentreptow (DE); SALZSIEDER, Eckhard, 17495 Karlsburg (DE); FROHN, Wolfgang, 95197 Schauenstein (DE); SCHULTZ, Christian, 17033 Neubrandenburg (DE)
(74) Vertreter: Hertin und Partner Rechts- und Patentanwälte PartG mbB
(86) Internationale Anmeldenummer: PCT/EP2018/069761
(87) Internationale Veröffentlichungsnummer: WO 2019/016367

(56) Entgegenhaltungen:
- WO-A1-2008/118549
- WO-A1-2013/191836
- WO-A2-2008/118543
- WO-A2-2012/001543
- GB-A- 2 221 620
- N.N.: "IRW-News: I-Minerals Inc.: I-Minerals Halloysit-Produkt Ultra Hallopure wird vom deutschen Diabetes-Institut Gerhardt Katsch Karlsburg in der Entwicklung von mineralimprägniertem Material zur Wundbehandlung verwendet", MARKETSCREENER , 18. Januar 2017 (2017-01-18), XP002785843, Gefunden im Internet: URL:https://de.marketscreener.com/I-MINERA LS-INC-21105521/news/IRW-News-I-Minerals-I nc-I-Minerals-Halloysit-Produkt-Ultra-Hall opure-wird-vom-deutschen-Diabetes-23710967 / [gefunden am 2018-10-18]
- N.N.: "Halloysite nanotubes incorporated into wound treatment cloth", NANOTECH , 18. Januar 2017 (2017-01-18), XP002785833, Gefunden im Internet: URL:http://www.nanotechmag.com/nanoclays-i ncorporated-wound-treatment-cloth/ [gefunden am 2018-10-18]
- DATABASE WPI Week 201741 Thomson Scientific, London, GB; AN 2017-28530S XP002785847, & CN 106 581 729 A (FUYANG HUIKANG MEDICAL INSTR CO LTD) 26. April 2017 (2017-04-26)
- DATABASE MEDLINE [Online] US NATIONAL LIBRARY OF MEDICINE (NLM), BETHESDA, MD, US; Dezember 2014 (2014-12), ALAVI MEHROSADAT ET AL: "The effect of a new impregnated gauze containing bentonite and halloysite minerals on blood coagulation and wound healing.", XP002785860, Database accession no. NLM25004023 & ALAVI MEHROSADAT ET AL: "The effect of a new impregnated gauze containing bentonite and halloysite minerals on blood coagulation and wound healing.", BLOOD COAGULATION & FIBRINOLYSIS : AN INTERNATIONAL JOURNAL IN HAEMOSTASIS AND THROMBOSIS DEC 2014, Bd. 25, Nr. 8, Dezember 2014 (2014-12), Seiten 856-859, ISSN: 1473-5733
- DATABASE WPI Week 201645 Thomson Scientific, London, GB; AN 2016-33627L XP002785865, & CN 105 561 370 A (ANHUI KESHENG BIOTECHNOLOGY CO LTD) 11. Mai 2016 (2016-05-11) -& DATABASE EPODOC [Online] EUROPEAN PATENT OFFICE, THE HAGUE, NL; XP002785866, Database accession no. CN-201510207517-A
- DATABASE WPI Week 201023 Thomson Scientific, London, GB; AN 2009-P79280 XP002785877, & CN 101 543 565 A (BU J) 30. September 2009 (2009-09-30)
- Mehrosadat Alavi ET AL: "The effect of a new impregnated gauze containing bentonite and halloysite minerals on blood coagulation and wound healing", Blood Coagulation and Fibrinolysis, 1 January 2014 (2014-01-01), pages 856-859, XP055855698, Retrieved from the Internet: URL:https://www.researchgate.net/profile/M -Ali-Okhovat/publication/263740674_The_eff ect_of_a_new_impregnated_gauze_containing_ bentonite_and_halloysite_minerals_on_blood _coagulation_and_wound_healing/links/59fe2 6a1458515d0706acb7f/The-effect-of-a-new-im pregnated-gauze-containing-bentonite-and-h alloysite- [retrieved on 2021-10-27]

## Beschreibung

Die Erfindung betrifft eine Wundauflage, die blutstillende, gerinnungsfördernde und biozide Eigenschaften hat und daher insbesondere für die Akutwundversorgung eingesetzt werden kann. Typische Anwendungsbereiche der Akutwundversorgung sind beispielsweise Versorgungen von Schussverletzungen. Die erfindungsgemäße neuartige Wundauflage weist insbesondere ausgewählte Halloysite auf, die beispielsweise auf textile Fasern oder Vliese aufgebracht werden.

### Stand der Technik

Im Stand der Technik sind mehrere Wundauflagen für Schussverletzungen bekannt. Viele Mittel, um Schussverletzungen zu behandeln umfassen beispielsweise Bandagen aus Baumwolle oder anderen Materialien. Des Weiteren gibt es sogenannte Mullbinden, die eine Träger-Gaze aufweisen, die Chitosan-beschichtet ist. Weiterhin sind blutstillende Verbände bekannt, die mit Kaolin beschichtet sind, die die Blutgerinnung beschleunigen. Neben Kaolin können zur Beschichtung von Verbänden auch Zeolithgranulate, Dreischichtsilikate oder Diatomeenerde verwendet werden. Bei der Herstellung von mineralbasierten Wundauflagen erfolgt das Aufbringen des Mineralstoffes des Substrates durch Techniken, wie das Tauchen oder Besprühen, gegebenenfalls unter Verwendung von Bindemitteln oder chemischen Additiven.

WO2008118543 beschreibt poröse Vorrichtungen mit funktionellen Partikeln (u.a. Halloysit) und interpenetrierendem Polymer-Netzwerk (z.B. Chitosan), sowie Halloysit enthaltende Wundauflagen mit antibiotischer und Blut-absorbierender Wirkung.

### Nachteile des Standes der Technik

Der beschriebene Stand der Technik weist zahlreiche Nachteile auf:
- hohe Temperaturentwicklungen (bis 70° C mit Verbrennungen im Wundumfeld) und schlechte Anwendbarkeit im Falle von Zeolithgranulaten und anderen Mineralgranulaten, sowie erforderliche Wundreinigung nach der Blutsstillung;
- Inhomogenitäten bei der Beschichtung oder Imprägnierung textiler Wundauflagen durch die bekannten Techniken und schlechte Ablösbarkeit von behandelten Wunden, sowie hohe Herstellungskosten durch Einsatz diverser Additive,
- fehlende Reinheitsanforderungen der im Stand der Technik verwendeten, natürlichen Mineralstoffe; die Mineralstoffe erfüllen nicht die Reinheitsanforderungen der Pharmacopeen und sind daher hinsichtlich der Medizinproduktzulassung als kritisch zu bewerten;
- mit den gegenwärtig bekannten Imprägnierungs- und Beschichtungstechniken wird nur eine vollflächige Imprägnation des textilen Substrates erzielt, was die schnelle Durchströmung behindern kann;

### Aufgabe der Erfindung

Es war die Aufgabe der Erfindung, eine Wundauflage bereitzustellen, die die Nachteile des Standes der Technik nicht aufweist. Insbesondere war es die Aufgabe der Erfindung, eine Wundauflage bereitzustellen, die die Merkmale der Blutstillung mit den Merkmalen des verminderten Thrombose-Risikos verbindet. Weiterhin weist die Aufgabe der Erfindung auf, dass sich die Wundauflage sehr flexibel an die Haut anlegt und nach der Akut-Wundversorgung wieder leicht von der Wunde lösbar ist und daher keine Neublutung verursacht. Die Wirksamkeit der Wundlauflage soll auch bei Patienten gegeben sein, die Blutverdünner-Medikamente einnehmen oder unabhängig hiervon eine verminderte Blutgerinnungsaktivität aufweisen.

### Lösung der Aufgabe

Die genannte Aufgabe wird gelöst durch eine hämostatische Wundauflage, die als Träger ein textiles Substrat, beispielsweise aus Verbundfasern und Halloysit aufweist, dadurch gekennzeichnet, dass die Wundauflage einen Halloysit-Anteil von 10 g/m² - 60 g/m² umfasst. Textile Substrate umfassen insbesondere Gewebe, Gewirke, Gestricke und/oder Vliese. Die Erfindung betrifft demgemäß eine neuartige Mineralimprägnierte textile Wundauflage, die überraschend gute blutstillende und biozide Wirkungen verbunden mit einem verminderten Thrombose-Risiko aufweist.

Die erfindungsgemäße Wundauflage lässt sich überraschend flexibel auf die Haut legen und ist nach der Akutwundversorgung sehr gut ablösbar und verursacht daher keine Neublutungen. Überraschender Weise kommt es bei der Verwendung der erfindungsgemäßen Wundauflage zu keiner Temperatur- und/ oder Keimbelastung der zu versorgenden Wunde.

Die Halloysite lassen sich überraschend gut homogen auf dem textilen Substrat verteilen, sodass eine gute und schnelle Blutaufnahme und Durchströmung des textilen Substrates bei der Versorgung von Wunden gewährleistet ist.

Es ist das Verdienst der Erfinder, gezeigt zu haben, dass die Verwendung von Halloysiten zu einer überraschend verbesserten Blutstillung insbesondere bei Schussverletzungen führt, wenn die Halloysite auf einem textilen Substrat in Form einer Wundauflage aufgebracht sind.

Es lag nicht nahe, dass diese Eigenschaften der verbesserten Blutstillung mit einem überraschend verminderten Thrombose-Risiko einhergehen.

### Bevorzugte Ausführungsformen der Erfindung

Die bevorzugten Ausführungsformen der Erfindung ergeben sich aus den Unteransprüchen.

In einer bevorzugten Ausführungsform ist vorgesehen, dass das textile Substrat, auf dem die Halloysite aufgebracht sind, Verbundfasern umfasst. Überraschender Weise sind Verbundfasern besonders gut geeignet, Halloysite gleichmäßig aufzunehmen bzw. besonders gut geeignet, um mit Halloysiten beschichtet zu werden. Es war völlig überraschend, dass die Kombination aus Verbundfasern und Halloysiten bzw. die Kombination aus Verbundfasern und der Beschichtung mit Halloysiten zu einer überraschend guten blutstillenden und bioziden Wirkung bei der Wundversorgung führt.

Die bisher eingesetzten Schichtsilikate Kaolinit und Montmorillonit sind blättchenförmig und monopolar (negative Schichtladungen) wodurch im Vergleich mit dem röhrchenförmigen und bipolaren erfindungsgemäßen Halloysit die Einwirkung auf die Verkürzung der natürlichen Gerinnungskaskade geringer ist.

Ein weiterer Vorteil ist es, dass die erfindungsgemäße Wundauflage beim Patienten als sehr viel schonender empfunden wird, als die Produkte, die es im Stand der Technik gibt. Aus diesem Grunde ist die Bereitschaft, die Wundauflage beispielsweise bei der Notfallversorgung anzuwenden, sehr viel größer, als dies bei den bekannten Produkten der Fall ist.

Bevorzugte Verbundfasern sind beispielsweise Materialen aus Viskose, Milchproteinfasern und/oder Algen.

Besonders bevorzugte Verbundfasern sind Regeneratfasern, beispielsweise Lyocell, Cupro, Viskose oder Modal.

Regeneratfasern sind bevorzugt Fasern, die insbesondere aus natürlich vorkommenden nachwachsenden Rohstoffen über chemische Prozesse hergestellt werden können. Insbesondere handelt es sich um Cellulosederivate beispielsweise aus Holz.

Bevorzugt können selbstverständlich auch synthetische Chemiefasern oder Naturfasern eingesetzt werden. Als Chemiefasern werden im Sinne der Erfindung alle Fasern bezeichnet, die künstlich, beispielsweise nach chemisch-technischen Verfahren aus natürlichen oder synthetischen Polymeren, sowie aus anorganischen Stoffen hergestellt werden können. Beispielsweise können synthetische Polymere wie Polyester, Polyamid, Polyimid, Aramid, Polyacryl und andere eingesetzt werden.

Weiterhin ist es auch möglich, Fasern aus natürlichen Polymeren, wie Acetat, Triacetat, Alginat, Proteinfasern, Chitin oder Elastodien zu verwenden. Bevorzugt sind auch Fasern aus regenerierter Cellulose. Bei den bevorzugten Viskosefasern handelt es sich um solche, die durch ein Viskoseverfahren aus Cellulose hergestellt werden. Die bevorzugten Modalfasern werden in einem modifizierten Viskoseverfahren hergestellt und haben deshalb eine höhere Festigkeit als Viskose im trockenen oder nassen Zustand. Lyocell wird in einem Nassspinnverfahren hergestellt, wobei die Fasern sich durch eine sehr hohe Trocken- und Nassfestigkeit auszeichnen. Bei den bevorzugten Milchproteinfasern kann es sich um lineare Makromoleküle mit mindestens 85 % Masseanteil Milchsäureestereinheiten handeln. Weitere bevorzugte Fasern sind solche, die aus Cellulosefasern oder aus Proteinfasern, wie z. B. modifizierte Sojabohnenproteinfasern, Zein, Caseinfasern oder künstliche Fasern, die beispielsweise durch Mikroorganismen erzeugt werden.

Es war völlig überraschend, dass Verbundfasern die Mischungen umfassen, die aus der Gruppe der Viskose-, Algen- und/oder Milchprotein-Fasern ausgewählt sind, besonders vorteilhafte Eigenschaften bei der Verminderung der Blutstillungszeiten haben. Weiterhin lag es für den Fachmann nicht nahe, dass diese bevorzugten Verbundfasern im Zusammenwirken mit den Halloysiten eine besonders gute biozide Wirkung haben und weiterhin die Nachteile des Standes der Technik nicht aufweisen. Zu diesen Nachteilen zählt insbesondere die als unangenehm empfundene Temperatur- und Hitzeentwicklung bei den Wundauflagen des Standes der Technik.

In einer weiteren besonders bevorzugten Ausführungsform werden beispielsweise auf den genannten Verbundfasern oder auf andere textile Substrate Halloysite in Form von Halloysit-Nanotubes aufgetragen. Überraschender Weise führt die Verwendung von Nanotubes zu besonders effizient einsetzbaren Wundauflagen. Die Verwendung von Nanotubes führt zu einer Wundauflage, die die Blutstillung besonders schnell und ohne Nachblutungen ermöglicht.

Bevorzugt werden insbesondere Halloysit-7Å- und Halloysit-10Å-Nanotubes eingesetzt. Insbesondere die bevorzugten Halloysit-Nanotubes eignen sich besonders überraschend, um das erfindungsgemäße Problem zu lösen. Diese überraschende Wirkung tritt auch dann auf, wenn nicht homogene Nanotubes der genannten bevorzugten Nanotubes verwendet werden, sondern Mischungen hieraus. Es kann beispielsweise bevorzugt sein, eine Halloysit-Nanotubes-Mischung zu verwenden, die sowohl Halloysit-7Å- und Halloysit-10Å-Anteile aufweist. Es lag für den Fachmann nicht nahe, dass die bevorzugten Nanotubes eine überraschend gute blutstillende Wirkungen haben, ohne dass sie die Nachteile des Standes der Technik aufweisen.

In einer bevorzugten Ausführungsform der Erfindung ist vorgesehen, dass die Halloysit-Nanotubes die folgenden Abmessungen aufweisen: Innerer Durchmesser: 10 bis 20 Nanometer, äußerer Durchmesser: 50 bis 70 Nanometer und/oder Länge: 0,3 bis 4 Mikrometer. Es war völlig überraschend, dass Halloysit-Nanotubes mit den besonders bevorzugten Abmessungen besonders gut geeignet sind, um das erfindungsgemäße Problem zu lösen.

Es ist bevorzugt, wenn die Halloysite bzw. das Halloysit-Material bei 200 ° Celsius vorab behandelt werden. Es war überraschend, dass die Wirkung der Wundauflage noch weiter überraschend verbessert werden kann, wenn die Halloysite bei einer Temperatur von 200 ° Celsius vorbehandelt werden, bevor sie auf das textile Substrat aufgebracht oder mit diesem getränkt werden.

Bevorzugter Weise werden durch die Behandlung bei 200 ° Celsius die Halloysit-10Å-Anteile in Halloysit-7Å- Anteile umgewandelt. Diese Umwandlung kann im Wesentlichen alle Halloysit-10Å-Anteile betreffen oder aber nur einen Teil von diesen. Der Fachmann kann, ohne erfinderisch tätig zu werden, anhand der Behandlungszeitdauer bestimmen, welcher Anteil von Halloysit-10Å-Anteilen in Halloysit-7Å-Anteile umgewandelt wird. Besonders bevorzugt kann vorher eine Wasser-Plasma-Behandlung eingesetzt werden, um das Zeta-Potential einzelner Halloysit-Röhrchen bzw. Halloysit-Nanotubes zu verringern und ihre Fähigkeit zur Dispersion zu verbessern.

Es war völlig überraschend, dass hierdurch neuartige textile Wundauflagen bereitgestellt werden können, die mittels verschiedener Drucktechnologien, insbesondere partiell mit Halloysiten oder Halloysitpasten aus den bevorzugten Nanotubes bedruckt werden können.

Es kann bevorzugt sein, die verwendeten Halloysit-Nanotubes durch Auswahl und Aufbereitungsverfahren an die Reinheitserfordernisse hinsichtlich von Nebenmengenanteilen (z. B. kristallines Silikat) und Schwermetallen anzupassen. Es war ebenfalls völlig überraschend, dass als mineralische Komponente die genannten Halloysit-Nanotubes eingesetzt werden können, da sie über den notwendigen Reinheitsgrad verfügen und bevorzugt mittels Drucktechnologien auf die textilen Fasern aufgebracht oder mittels verschiedener Vliesherstellungsverfahren, bevorzugt mit dem Spunlace-Verfahren in die textilen Substrate direkt inkorporiert werden. Für den Prozess des Bedruckens wird in einer bevorzugten Ausführungsform eine Mineral-Wassersupension mit einem geringen Anteil eines Acrylat-, Tylose- und/ oder Gelatinebinders hergestellt.

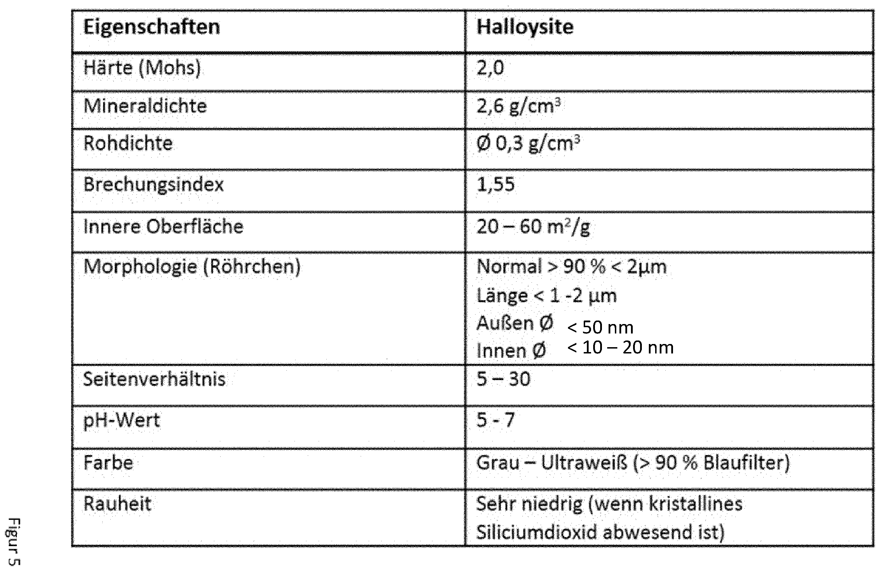

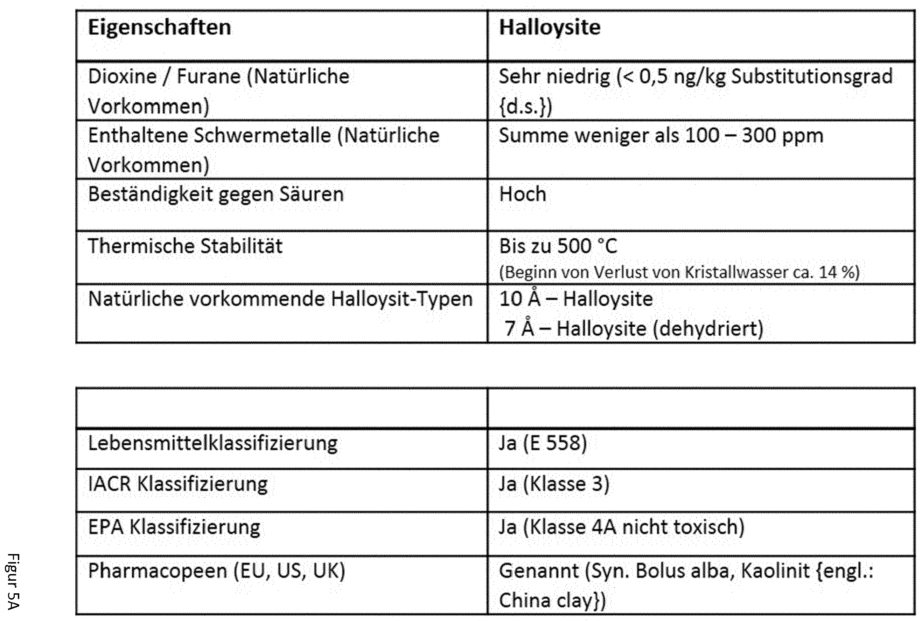

Die Siebdrucktechnologie ermöglicht im Gegensatz zu bekannt Sprüh- und Tauchtechnologien des Standes der Technik eine exakte und variierende Imprägnation der textilen Gewebefläche, sodass bevorzugt vollständig auf die Zugabe weiterer Chemikalien und Agentien, wie dies im Stand der Technik vorgesehen ist, verzichtet werden kann. Die Halloysit-Nanotubes sind biokompatibel, und unterliegen nicht der EU-Nanopartikeldefinition (> 50 % < 100 nm) und sind von der EPA als non toxic/ GRAS eingestuft. Aus der Fig. 5A kann die bisher erfolgte umfassende Klassifizierung und Zuordnung zu Einsatzmöglichkeiten von Halloysiten entnommen werden.

In einer bevorzugten Ausführungsform können die Halloysite bzw. die Halloysit-Nanotubes mittels eines Bindemittels auf das Gewebesubstrat aufgebracht werden. Auch wenn es zunächst naheliegend erscheint, ein Bindemittel zu verwenden, um die Halloysite auf dem textilen Substrat mit einem Bindemittel zu positionieren und zu fixieren, war es überraschend, dass die vorteilhafte Wirkung der Halloysite durch das Bindemittel noch einmal verbessert werden kann.

Die erfindungsgemäßen textilen Fasern bzw. die Wundauflagen stellen daher innovative Entwicklungen dar. Sie basieren insbesondere auf Viskose/Algen- und/oder Viskose/ Milchproteinfaser-Kompositen, die zusätzlich bis zu 30 % Halloysit-Nanotubes direkt enthalten können. Untersuchungen haben die überraschend guten bioziden Eigenschaften solcher textilen Fasern belegt, was im Hinblick auf ein breites Spektrum zu versorgender Wunden sehr vorteilhaft ist. Ferner wurde durch Untersuchungen festgestellt, dass eine geringere Vliesdichte als bei den Textilien des Standes der Technik (z.B. Z-Medica) die Blutstillungszeiten verkürzt.

In einer besonders bevorzugten Ausführungsform ist die Wundauflage dadurch gekennzeichnet, dass folgende Fasern alternativ oder als Gemisch zur Herstellung der Auflage verwendet wurden:
a) Lyrocellfasern mit bis zu 20 % (Vol-%) Algenzusatz (bevorzugt in die Faser inkorporiert)
b) Gemisch aus Viskosefaser und Milchsäurefaser (bevorzugt 50:50)
c) Milchproteinfaser mit bis zu 30 % Halloysit-Anteil (bevorzugt in die Faser inkorportiert)
d) Milchproteinfasern (bevorzugt 100 %).

Bevorzugt ist es hierbei, dass der in die Fasertypen inkorporierte Halloysit-Anteil durch ein Verfahren im Rahmen des Faserherstellungs- und/oder Anspinnprozesses noch vor dem Extrudieren bereits in die Spinnmasse dispergiert wird und dadurch in die Matrix aus der Spinnmasse entstehenden Fasern eingebunden wird.

Besonders bevorzugt ist es, dass die Fasern anschließend zu Vliesstoff, Gewebe, Gaze, Gestrick, Gewirke oder Gelege weiter bearbeitet werden, um das textile Substrat zu binden.

Ganz besonders bevorzugt ist es hierbei, dass auf das textile Substrat mittels eines Flachdruckverfahrens eine aus Halloysit-Nanotubes, destilliertem Wasser und bei Verwendung eines medizinisch unbedenklichen Binders auf Basis einer Zellulose-Gelatine-Komposition mit maximal 5 % Volumenanteil hergestellte Paste auf das Substrat aufgedruckt und somit auf dieses fixiert wird, ohne dass es eines anschließenden Absaugens, Abschwemmens, oder Abschüttelns des nicht auf der Substratoberfläche fixierten Materials bedarf.

Ganz besonders bevorzugt ist es, dass nicht die gesamte Substratoberfläche mit der Halloysit-haltigen Paste bedruckt wird, sondern ein Flächenanteil von 30 bis 70 % des Substrates.

Insbesondere hat das mit Halloysit beladene Substrat ein Gesamtgewicht von bevorzugt 25 bis 145 g/m², vorzugsweise von 50 bis 80 g/m².

Nach der Konfektionierung und Verpackung kann eine Sterilisierung mit GammaStrahlung oder Ethylengas-Behandlung erfolgen.

Die erfindungsgemäßen Wundauflagen können je nach Verwendungszweck zusätzlich beispielsweise synthetische und/oder natürliche Polymere, beispielsweise Polyacrylate, antibakterielle Substanzen wie Jod, Silber, Kupfer und Zink oder Gemischen daraus, Naturkautschuke, Chitosane, Alginate, Hydrogele, Hydrokolloide und/oder Polyurethane enthalten. Zur Verstärkung der desinfizierenden Wirkung und/oder je nach Anwendungszweck können sie des Weiteren insbesondere oxidierende anorganische und/oder organische Verbindungen, desinfizierende chemische Verbindungen, übliche Füll- und Hilfsstoffe, Duftstoffe und/oder Farbstoffe enthalten. Erfindungsgemäß finden die beschriebenen Halloysit-basierten Auflagen Verwendung zur Herstellung von wundstillenden Materialien, insbesondere Kompressen, Pflaster und/oder Verbandmaterial.

Die bevorzugten Wundauflagen, die bis zu 30 Masse-% Halloysit-Nanotubes enthalten können und auf Viskose/Algen- und/oder Viskose/Milchproteinfaser-Kompositen aufgebracht sind, eignen sich überraschend gut für die Erstversorgung von Schusswunden. Aber auch Verletzungen mit Stichwaffen können sehr effizient behandelt werden. **Bevorzugt gilt bei den Faserzusammensetzungen Vol.-% und bei den Pastenzusammensetzungen Masse-%.**

Die Konfektionierung der beschriebenen blutstillenden Wundauflage ist überraschend einfach und variabel zu realisieren, sodass für verschiedenste Anwendungen wie Notfallversorgung, Erste Hilfe oder chirurgische Krankenversorgung maßgerechte Konfektionierungen dargestellt werden können. Neben den besonderen und überraschenden blutstillenden und bioziden Wirkungen der erfindungsgemäßen Wundlauflage ist die überraschend einfache Konfektionierung ein weiterer Vorteil dieser Vorrichtung. Bei der Wundversorgung ist es von großer Wichtigkeit, dass die Auflagen zur Versorgung einer Wunde entsprechend der Größe dieser konfektioniert werden können. Dies ist insbesondere dann wichtig, wenn die betreffende Person sich noch bewegen muss, oder das Wundareal Bewegungen ausgesetzt ist. Da die Wundauflage in diesen Fällen die Bewegung zulassen muss, ist es wichtig, dass die Wundauflage als Wundbehandlungsmittel auf einfachem Wege konfektioniert werden kann. Insbesondere bei der Verwendung im Feld oder allgemein in Krisengebieten ist es wichtig, dass in sehr kurzer Zeit eine maßgerechte Konfektionierung bereitgestellt wird.

Wundbehandlungsmittel im Sinne der Erfindung können die Halloysit-Textilsubstrate in Form einer Wundkompresse mit einer porösen Fläche sein, welche auf die Wunde gelegt wird. Die Kompresse kann entweder Bestandteil eines Pflasters, eines Verbandmaterials, einer Kompresse oder lose sein. Ebenso kann das Wundbehandlungsmittel auf andere Weise auf Kompressen, Pflaster oder Binden aufgebracht oder darin enthalten sein oder Bestandteil eines Verbandmaterials sein. Diese Behandlung kann am Menschen oder am Tier erfolgen. Darüber hinaus stellen Notfallversorgungskits bei Schussverletzungen, Einwegwindeln bei blutenden Patienten, Damenbinden oder Stilleinlagen bevorzugte Ausführungsformen der Erfindung dar. Wundstillende Materialien im Sinne der Erfindung können ferner beispielsweise Pasten oder poröse oder nichtporöse Materialien wie Filme, beispielsweise Siloxanfilme, Polyesterfilme, Polyurethanfilme sein. Das Material zur Anwendung bei der Behandlung von Schusswunden ist bevorzugt dadurch gekennzeichnet, dass das Wundbehandlungsmittel wundseitig von einem porösen textilen Faserstoff, insbesondere einem Vlies, Gewirk oder Gestrick sowie mit einer feuchtigkeitsdurchlässigen Folie, abgedeckt ist, welche auch antiseptische Mittel umfassen können.

Die Wundauflage gemäß der Erfindung kann bevorzugt auch ganz allgemein als Verbandmittel eingesetzt werden. Hiermit bezeichnet man im Sinne der Erfindung jene Materialien, aus denen ein medizinischer Verband hergestellt wird. Sie werden deshalb auch als Verbandmaterial oder umgangssprachlich Verband(s)zeug bezeichnet. Sie werden oft in Form von Verbandkästen griffbereit gelagert. In weiterer Folge lässt sich zwischen sterilem oder zumindest keimarmen Wundauflagen und nicht sterilem Befestigungsmaterial unterscheiden. Ebenfalls bevorzugt sind auch gebrauchsfertige, kombinierte Zusammenstellungen von Wundauflage und Befestigungsmaterial. Sterile Wundauflagen sind Kompressen und Verbandstücher. Zum Befestigungsmaterial gehören bevorzugt Mullbinden, Idealbinden, elastische Fixierbinden, Trikotschlauchbinden und Dreiecktücher.

Gebrauchsfertige Zusammenstellungen sind bevorzugt Verbandspäckchen und Wundschnellverbände.

In einer weiteren bevorzugten Ausführungsform ist vorgesehen, dass das textile Substrat eine Dichte zwischen 30 g/ m² und 120 g/ m², bevorzugt 50 g/ m², aufweist. Überraschender Weise führt die bevorzugte Dichte zu einer besonders schnellen Gerinnung und Unterbindung des Blutes in der Wunde. Es war völlig überraschend, dass die genannten Parameterbereiche dazu führen, dass die erfindungsgemäße Aufgabe besonders gut gelöst wird.

In einer weiteren bevorzugten Ausführungsform ist vorgesehen, dass die Verbundfasern zwischen 3 % und 30 % (Vol-%) Algenanteil aufweisen und/ oder bis zu 100 % aus Milchprotein bestehen und alternativ einen in den Fasern beinhalteten Halloysit-Anteil von bis zu 30 % aufweisen. Der bevorzugte Anteil an Verbundfasern führt zu einer besonders guten Lösung der erfindungsgemäßen Aufgabe. Es ist besonders vorteilhaft, dass diese bevorzugten Ausführungsformen nach der Akutversorgung sehr einfach und problemlos von der Wunde entfernt werden können, sodass es zu keinen Nachblutungen kommt. Des Weiteren ist es ein Vorteil dieser bevorzugten Ausführungsform, dass sie überraschend wirkungsvoll bei Patienten eingesetzt werden kann, die blutverdünnende Mittel einnehmen.

Die Erfindung betrifft in einem Aspekt daher eine hämostatische Vorrichtung (ein Hämostatikum) umfassend (a) ein textiles Substrat von Verbundfasern aus Mischungen aus Viskose, Algen und/oder Milchprotein (b) ein Temperaturbehandeltes Halloysit und/oder (c) ein Bindemittel zur Fixierung der Halloysite auf dem textilen Substrat. Durch Routineversuche kann der Fachmann diese Bestandteile so auswählen und kombinieren, dass bei in Kontaktkommen mit dieser Vorrichtung mit Blut eine Gerinnung verursacht wird und das Bluten einer Wunde in bevorzugt weniger als 2 Minuten unterbunden wird.

In einer anderen bevorzugten Ausführungsform ist vorgesehen, dass das Bindemittel 90 % bis 95 % deionisiertes Wasser und 5 % bis 10 % Acrylat umfasst. Vorteilhafter Weise erlaubt dies, das behandelte Halloysit-Material besonders gut an das Substrat zu binden bzw. es auf dem Substrat zu fixieren. Es war überraschend, dass hierfür keine weiteren Zusätze benötigt werden. Die bevorzugte Wundauflage stillt Blutungen in der Chirurgie, bei der Ersten Hilfe, bei der Notfallversorgung oder bei der Versorgung chronischer Wunden besonders effizient. Es lag für den Fachmann nicht nahe, dass die Wundauflage gemäß der bevorzugten Ausführungsform zu einer besonders guten Versorgung einer akuten oder chronischen Wunde führt.

In einer weiteren bevorzugten Ausführungsform deckt das Bindemittel und/oder das Halloysit-Material nur einen Teil der textilen Substratoberfläche bzw. der Wundauflage ab. Bevorzugt werden 30 % bis 70 % der Substratoberfläche abgedeckt; bevorzugt werden Bindemittel und/oder Halloysit-Material auf dem Substrat mit Hilfe eines Druckverfahrens (z.B. Siebdruck) aufgebracht. Es ist bevorzugt, wenn die Halloysite 30 bis 70 % der Oberfläche des textilen Substrates abdecken, da in diesem Falle bei der akuten wie auch bei der chronischen Wundversorgung die Keimbelastung der Wunde überraschend reduziert werden kann. Insbesondere bei der Versorgung chronischer Wunden ist es wichtig, die Keimbelastung zu reduzieren. Die biozide Wirkung der bevorzugten Wundauflage führt dazu, dass diese sowohl bei der Akutversorgung von Wunden wie auch beispielsweise in der Altenpflege bei der Versorgung von chronischen Wunden äußerst effizient zur schnellen Blutstillung und zur Verhinderung von Nachblutungen eingesetzt werden kann.

Erfindungsgemäß umfasst die hämostatische Vorrichtung einen Anteil zwischen 10g/m² und 60 g/m² des Halloysit-Materials, welches bevorzugt einer schnellen Trocknung bei 130 °C ausgesetzt wurde. Überraschenderweise ergibt diese Prozedur ein hochflexibles und wasserfestes Substrat. Insbesondere bei der akuten Notversorgung ist es wichtig, eine wasserfeste Wundauflage einsetzen zu können. Die Wundauflagen des Standes der Technik sind bei Unfällen im Freien oder bei Schussverletzungen nur mit Nachteilen einsetzbar.

Unfälle ereignen sich oft im Zusammenhang mit Unwettern oder Überschwemmungen. Die Wundauflagen des Standes der Technik durchnässen in diesen Fällen häufig und verlieren so ihre Wirkung. Sie eignen sich dann weder dauerhaft zur Blutstillung noch weisen sie eine biozide Wirkung auf. Überraschender Weise ist die bevorzugte Wundauflage sehr gut in Unwetter- und Krisensituationen einsetzbar.

Bevorzugt ist es, dass die hämostatische Vorrichtung oder die Wundauflage unter Einsatz von behandeltem Halloysit-Material hergestellt wird, welches steril und bevorzugt im Einklang mit den Arzneibuch-Kriterien für Kaolinum Ponderosum/medizinischem Kaolin ist.

Es ist weiterhin bevorzugt, wenn das Halloysit-Material bei 200 °C behandelt wurde, um bevorzugt Halloysit-10Å-Anteile vollständig in Halloysit-7Å-Röhrchen umzuwandeln. Zusätzlich kann vorher eine Wasser-Plasma-Behandlung eingesetzt werden, um das Zeta-Potential einzelner Halloysit-Röhrchen zu verringern und ihre Fähigkeit zur Dispersion zu verbessern. Bevorzugt wird Halloysit-Material verwendet, welches nur einen Anteil von weniger als 1 % oder im Wesentlichen gar keine Anteile inerter mineralischer Substanzen wie Quarz, Cristobalit, Feldspat, Alunit oder Glimmer aufweist, welche häufige Verunreinigungen darstellen. Die Blutung wird überraschenderweise auch dann gestillt bzw. das Blut absorbiert, wenn das Halloysit-Material räumlich nicht zu weit von der Wunde getrennt ist, so beispielsweise durch ein Vlies, das zwischen dem Halloysit-Material und der Wunde liegt. Ein besonderer Vorteil liegt also darin, dass die Wunde so nicht durch das Halloysit-Material verkrustet wird, sondern sauber bleibt. Typische Situationen, in denen ein solches bevorzugtes Wundbehandlungsmittel Anwendung findet, sind solche, in denen ein gewöhnliches Verbandsmaterial wie z.B. ein Pflaster ungünstig oder unerwünscht wäre, etwa bei Personen oder bei Tieren, die auf den Klebstoff allergisch sind etc. Außerdem ist es günstig, wenn die Blutung sehr schnell gestoppt werden muss, oder wenn die Wunde infiziert sein könnte, etwa bei Bissverletzungen durch Tiere oder bei Unfällen im Sport oder bei einer Verletzung durch Schuss- oder Hieb- bzw. Stichwaffen.

In einer weiteren bevorzugten Ausführungsform der Erfindung ist vorgesehen, dass die Halloysite beispielsweise als Halloysitpaste oder als ein anderes bereitgestelltes Material insbesondere mittels Siebdruck, Hochdruck, Tiefdruck oder Flachdruck auf dem textilen Substrat aufgebracht werden. Bevorzugt ist das Sieb- oder Flachdruckverfahren.

### Beispiele - spezielle Beschreibung der Erfindung

Im Folgenden soll die Erfindung anhand von Beispielen und Illustrationen näher erläutert werden, ohne auf diese beschränkt zu sein.

### Abkürzungserläuterungen

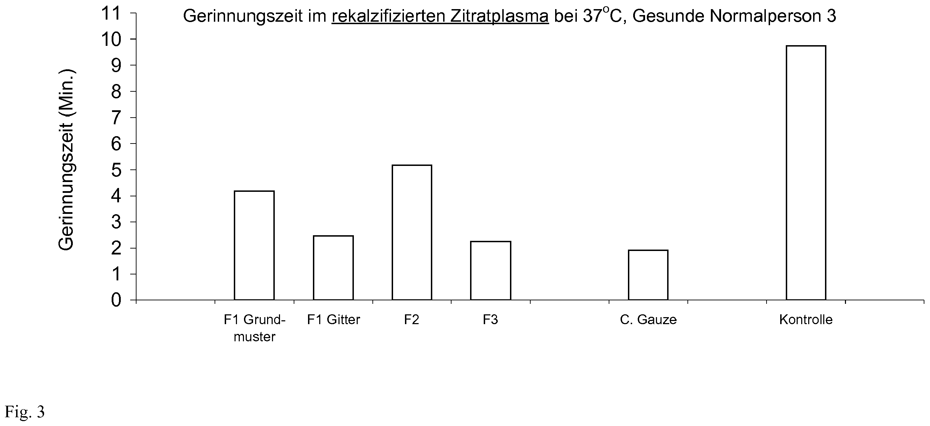

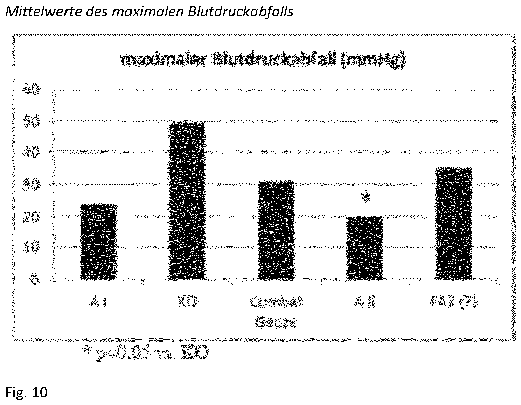

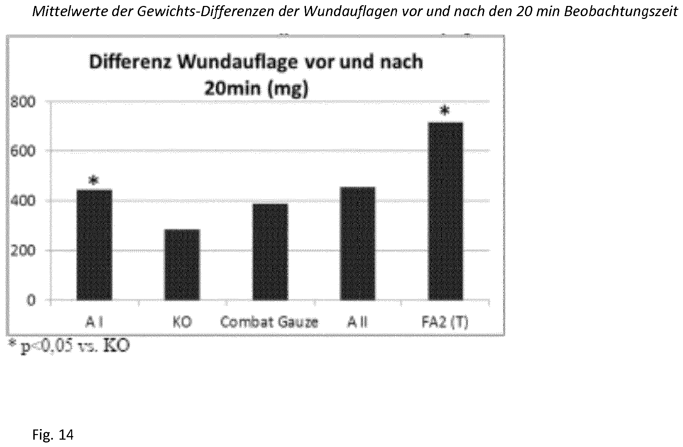

Abb1.:
   PES-V1:
      - Textil Polyethersulfon unbehandelt
      - Textil Polyethersulfon nur mit Acrylatbinder bedruckt
      - Textil Polyethersulfon mit Binder und Halloysittyp MF7(60gr/m²), vollflächig bedruckt
   PES-V2:
      - Textil Polyethersulfon unbehandelt
      - Textil Polyethersulfon nur mit Acrylbinder bedruckt
      - Textil Polythersulfon mit Binder und Halloysittyp MF7(30gr/m²), vollflächig bedruckt
   Cotton:
      - Baumwolle unbehandelt
      - nur mit Tylosebinder bedruckt
      - mit Tylosebinder und HNT MF7 nach Cu-Coating im NT-Plasma bedruckt,
         (biozid 30: 30gr/m² ; biozid 60: 60gr/m²) vollflächig bedruckt
   Kontrolle: natürliche Gerinnungzeit ohne Wundauflage
Abb2.:
   Cotton mit Tylosebinder und unterschiedlichen natürlichen HNT-Typen MF2,MF4
      (ungetempert) und MF4/200(bei 200°C getempert), alle 50gr/m², vollflächig
      bedruckt
   Kontrolle: natürliche Gerinnungszeit ohne Wundauflage
Abb3. und 4.:
   - Cotton mit Tylosebinder und HNT MF7 (30gr/m²) mit unterschiedlichen
      Druckmustern(F1 - F3), wodurch ein variierendes Verhältnis von bedruckter
      und unbedruckter Fläche entsteht ( siehe Foto)
   - C.Gauze: Produkt der Firma Z-Medica, U.S.
   - Kontrolle: natürliche Blutgerinnung ohne Wundauflage
Abb6.:
   - FA1:Textil Aramid ohne Binder und HNT MF7-Pastenbedruckung(130gr/m²) vollflächig bedruckt (nicht erfindungsgemäß)
   - FA2:Textil Aramid ohne Binder und HNT MF7-Pastenbedruckung(50gr/m²) vollflächig bedruckt
   - FV1a: Viskosevlies mit Acrylatbinde(10%) mit HNT MF7(50gr/m²) 70% bedruckt
   - FV1b: Viskosevlies mit Acrylatbinder(10%) mit HNT MF7(30gr/m²) 60% bedruckt
   - FV2: Viskoseflies mit 4% Algenzusatz mit Binder(5%) mit HNT MF7(30gr/m²) vollflächig bedruckt
   - FV3 Vliesmischung aus Viskose- und Milchsäureproteinfaser mit **Binder(5%)** mit HNT MF7(30gr/m²), vollflächig bedruckt
   - Combat Gauze: Produkt der Firma Z-Medica,U.S.
   - Kontrolle: natürliche Blutgerinnung ohne Wundauflage
Abb7.:
   - FV1/5: Lyocellvlies mit 5% Binder und HNT MF7(30gr/m²) vollflächig bedruckt
   - FV1/10a: Lyocellvlies mit 10% Binder(Tylose) und HNT MF7(30gr/m²) mit Muster
      Bedruckt (ca.70% der Fläche)
   - FV1/10b: Lyocellvlies mit 10% Binder(Acrylat) mit HNT MF7(30gr/m²) mit Muster
      bedruckt (60% der Fläche)
   - FA2/5a:Aramid mit 5% Acrylatbinder mit HNT MF7(30gr/m²) 70% bedruckt
   - FA2/5b: Aramid mit 5% Acrylatbind. mit HNT MF7(50gr/m²) 60% bedruckt
   - FM1: Viskosevlies mit Zinkoxid(16%) mit 5% Binder mit HNT MF7(30gr/m²) vollfl.
      bedruckt (vfb)
   - FM2: Viskosevlies mit Zinkoxid(8%) mit 5% Binder mit HNT MF7(30gr/m²) vfb
   - FM4: Vlies(Aramid 50%,Viskose 42%,Zinkoxid 8%) mit 5 %Binder mit HNT MF7
      (30gr/m²) vfb
   - FM5: Vlies(Viskose 96%,Algen 4%) mit 5% Binder mit HNT MF7(30gr/m²) vfb
   - FM8: Aramid als Gewirke mit 5% Binder mit HNT MF7(30gr/m²) vfb
   - Combat G.: Produkt der Firma Z-Medica,U.S,
Abb8./Abb9.:
   - Quikclot: Produkt der Firma Z-Medica,U.S
   - f-speed: Lyocell mit 5% Binder mit HNT MF7(30gr/m²) mit teilflächiger
      Bedruckung(ca.60%)
   - MF4: Lyocell mit 5% Binder mit HNT MF4(30gr/m²) vollfläch. Bedruckt
   - Kontrolle: natürliche Blutgerinnung ohne Wundauflage
Abkürzungserklärungen für tierexperimentelle Versuche (Abb. 10 - 14)
KO: Kontrolle Textil Cotton ohne jede Bedruckung
AI:Cotton mit 10% Binder mit HNT MF7(30gr/m²) vollflächig bedruckt
AII:Cotton mit 5% Binder mit HNT MF7(30gr/m²) vollflächig bedruckt
FA2(T): Cotton mit 10% Binder mit HNT MF7(60gr/m²) Cu-beschichtet im NT-Plasma vollflächig bedruckt
Combat Gauze: Produkt der Firma Z-Medica,U.S.

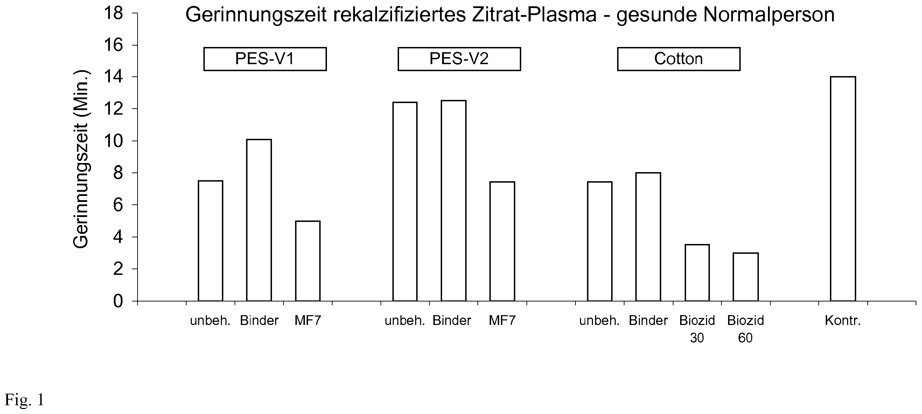

Figur 1 zeigt den Effekt von mit Halloysit-Nanotubes und Bindern imprägnierten Textilunterlagen auf die Gerinnungszeit im rekalzifierten Zitrat-Plasma eines repräsentativen gesunden Blutspenders im Vergleich zu unbehandelten Ausgangsmaterialien nur mit Bindern versehenen Textilien, sowie den zusätzlich mit Biozid 30 bzw. Biozid 60 imprägnierten Unterlagen aus Cotton. Biozid 30 und Biozid 60 sind Angaben, die sich auf Halloysitmaterial beziehen, welches mittels NT-Plasmatechnologie mit Kupfer beschichtet wurde. Hierbei zeigt sich die überraschend gute Wirkung der mit Halloysit-Nanotubes und Bindern imprägnierten Textilunterlagen auf die Gerinnungszeit.

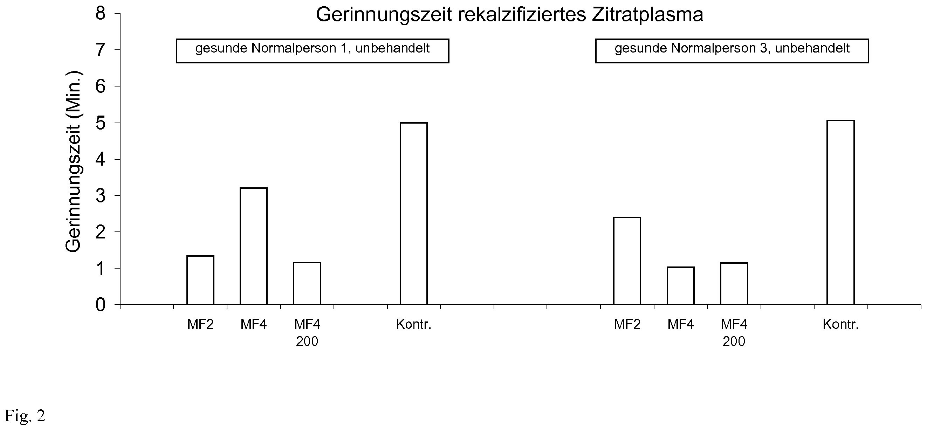

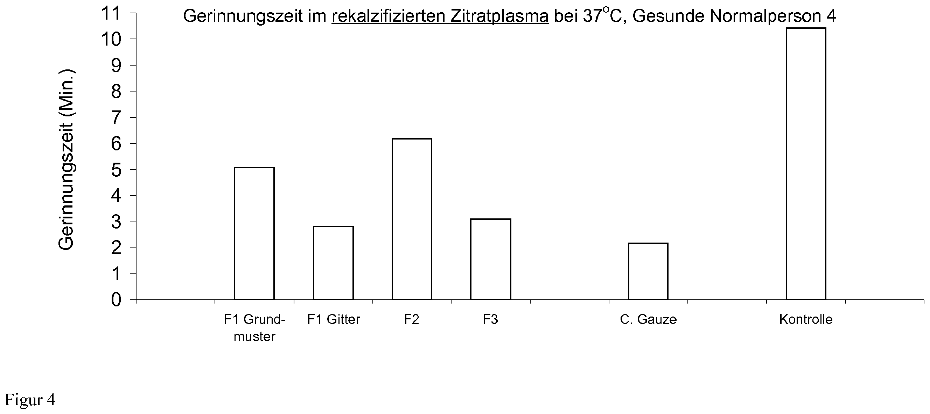

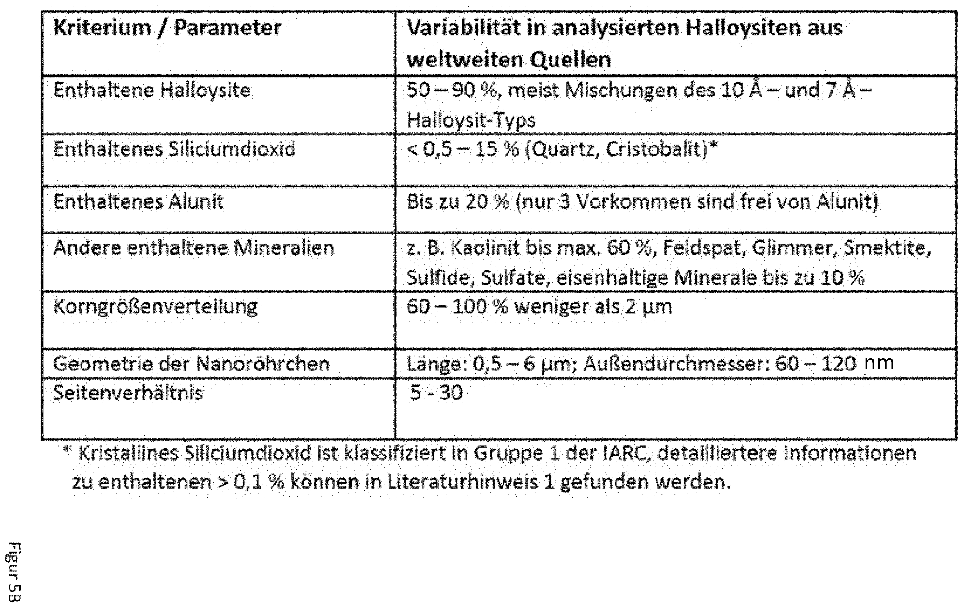

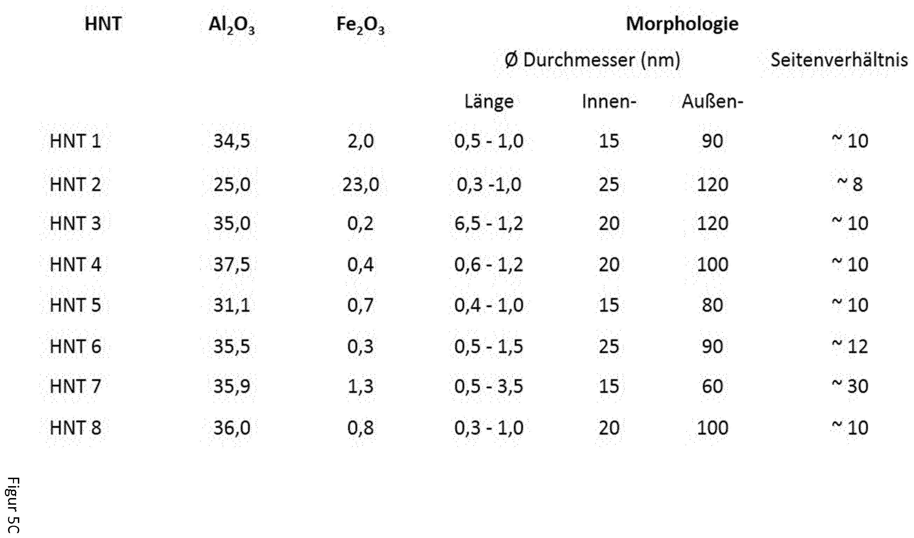

In Figur 2 wird die Testung neuer Halloysit-basierter Mineralien auf die Gerinnungszeit im rekalzifierten Zitrat-Plasma von zwei repräsentativen gesunden Blutspendern gezeigt. Hallyosit-basierte Mineralien haben einen deutlichen Einfluss auf die Gerinnungszeit. Je nach Behandlung der Halloysit-basierten Mineralien kann diese Wirkung in bestimmten Fällen noch einmal verbessert werden. Die Behandlung der Halloysit-basierten Mineralien kann beispielsweise eine 200 ° C-Behandlung sein. In Figur 3 wird die Testung neuer Muster von Mineral-imprägnierten Textilunterlagen (Fa. FROHN) auf die Gerinnungszeit im rekalzifeirten Zitrat-Plasma an gesunden Blutspendern (bei 37° C) im Vergleich zum unbeschichteten Grundmaterial und zu Produkten des Standes der Technik (Combat Gauze) gezeigt. Die erfindungsgemäßen Mineral-imprägnierten Textilunterlagen zeigen gegenüber dem unbeschichteten Grundmaterial eine überraschend verbesserte Wirkung auf die Gerinnungszeit. Obwohl die Mineral-imprägnierten Textilunterlagen gemäß der Erfindung viele Nachteile des Standes der Technik nicht aufweisen, erreichen sie die positiven Wirkungen von besonders wirkungsstarken bzw. von besonders guten Konkurrenzprodukten des Standes der Technik, ohne deren Nachteile zu haben. Die vorteilhaften Ergebnisse ließen sich anhand von verschiedenen Patienten (siehe Figur 4) verifizieren. Bevorzugte Eigenschaften der verwendeten Mineralien zum Imprägnieren der Textilunterlagen werden in den Figuren 5 (A - C) gezeigt.

Im Folgenden wird ein Beispiel für die Herstellung einer bevorzugten Wundauflage offenbart. Bei der Herstellung der bevorzugten Wundauflage war es die Aufgabe fein gemahlenes mineralisches Halloysit auf Vlies (non-woven) zu applizieren
• Technologie: Herstellung einer druckfähigen, wasserlöslichen Paste. Aufbringen der Paste auf das textile Substrat im Siebdruckverfahren. Anschließend Trocknung der applizierten Paste.
• Siebdruck
o Schablonenspezifikation
Herstellerbezeichnung: Saatilene HI-R / PE AM 34.100
PW
Monofiles Polyestergewebe, Leinenbindung
Technische Spezifikationen des Schablonengewebes:

| | |
|---|---|
| Anzahl der Fäden pro cm | : 34 |
| Fadendurchmesser | : 100 µ |
| Maschenweite | : 185 µ |
| Offene Fläche | : 43 % |
| Gewebedicke | : 173 µ |
| Theoretisches Farbvolumen | : 71 cm³ / m² |
| Spezifischer Querschnitt | : 0,267 mm² / cm |
| Druckart | : dot-Print |

o Maschinenspezifikation
Herstellerbezeichnung: BUSER Hydromag F7 2100/20/08
Mit gasbetriebenem Trockner F 128.2010G
Technische Spezifikation der Anlage:

| | |
|---|---|
| Maximale Druckbreite cm | : 1.700 mm |
| Anzahl verw. Druckwerke | : 1 |
| System Siebdruck | : Flachdruckschablone |
| Trocknertemperatur | : ca. 120 ° C |
| Produktionsgeschwindigkeit | : ca. 8 lfm/min |
| Rakelgeschwindigkeit | : ca. 1,2 m/s |

• Pastenrezeptur
o Stammansatz

| | |
|---|---|
| Wasser | : 900 - 950 gr |
| Binder | : 50 - 100 gr |
| Mit dem Zusatz eines Markers zur Rezeptur können Nachahmer-Produkte identifiziert werden. | |
| Summe | : 1000 g |
| Halloysit vorsichtig und unter Vermeidung jeglicher Klumpenbildung in den Stammansatz einrühren bis die entsprechende Pastenviskosität (63 dPas nach Haake VT02) erreicht wird. | |
| Einsatzmenge Halloysit Richtwert: ca. 250 bis 300 g | |

### Beispiele für blutstillende Wirkungen der erfindungsgemäßen Wundauflage

### In vitro Messungen der blutstillenden Wirkung Mineral-imprägnierter textiler Wundauflagen

### 1. Versuchsmethodik

Als Blutspender für die in vitro Untersuchungen wurden gesunde Normalpersonen und Patienten herangezogen, die aufgrund eines erhöhten Thromboserisikos aus klinischer Indikation mit Thrombozytenaggregationshemmer oder Vitamin K-Antagonisten behandelt wurden. Die Blutproben wurden durch Punktion der Vene in der Armbeuge unter Verwendung des Vacutainer-Systems der Fa. Becton Dickinson (USA) entnommen. Die Blutproben wurden in Natriumcitrat- und Serumröhrchen aufgenommen, die ebenfalls von der Fa. Becton Dickinson bezogen wurden. Die für die Testungen benötigten Plasmaproben wurden nach Zentrifugation aus den Natriumzitratröhrchen abpipettiert und in 50 ml Zentrifugenröhrchen überführt. In den Testreihen wurden sowohl frisch gewonnene als auch bis zu 6 Monate bei -70°C gelagerte Plasmaproben eingesetzt.

Zur Messung des Einflusses Mineral-imprägnierter Wundauflagen wurden 1 cm² große Stücke aus den Testmustern zugeschnitten und zusammen mit 1 ml Zitratplasma in 12 x 75 mm Teströhrchen aus durchsichtigem Kunststoff und mit Rundboden überführt. Nach Equilibrierung über 10 Min. bei 37°C im Wasserbad wurde die Gerinnung durch Zugabe von 500 ml einer frisch hergestellten 25 mmol/l CaCl₂-Lösung zur Rekalzifizierung des Plasmas in Gang gesetzt. Während der nachfolgenden Inkubation bei 37°C wurden die Proben mit Plastikrührstäbchen in kurzen Abständen durchmischt. Als Gerinnungszeit wurde auf der Stoppuhr der Zeitpunkt abgelesen, an dem in den entsprechenden Teströhrchen eindeutig ein Gerinnsel mit Pfropfbildung erkennbar war. Da die Gerinnung in den Ansätzen mit den unterschiedlichen Testmustern z.T. in kurzen Abständen einsetzte, wurden von jedem Untersucher gleichzeitig nicht mehr als 3 Teströhrchen bearbeitet.

Für die Messungen im Vollblut wurden aufgrund der in den Serumröhrchen sofort beginnenden Gerinnung 1 ml-Aliquote unmittelbar nach der Blutabnahme in die Probenröhrchen mit den Testmustern Mineral-imprägnierter Wundauflagen überführt. Die weitere Handhabung der Testansätze mit den Vollblutproben und die Bestimmung der Gerinnungszeit erfolgte in gleicher Weise wie in den oben beschriebenen Testansätzen mit rekalzifiziertem Zitrat-Plasma. In Vorversuchen zur Ermittlung der am besten wirkenden Mineralien wurden diese vor der Textilimprägnierung in Pulver- oder Granulatform unter den gleichen Bedingungen getestet und den Plasma- und Vollblutproben in einer Konzentration von 100 mg pro Milliliter zugesetzt.

### 2. Eingesetzte Materialien

Für die Blutentnahmen wurden, wie im Abschnitt Versuchsmethodik erwähnt, das Vacutainer-System der Fa. Becton Dickinson mit je 7 ml fassenden Natriumzitrat- und Serumröhrchen des gleichen Herstellers verwendet.

In Pulver- oder Granulatform wurden folgende Mineralien eingesetzt: MF2, MF4, MF4/200, MF7, QuikClot, f-speed und Kaolinit.

Die mit und ohne Mineralimprägnierung eingesetzten Textilmuster umfassten: PES-V1, PES-V1-MF7, PES-V2, PES-V2-MF7, Cotton, Cotton-MF7, Vokano, 1764 natur, 1764 weiß, 5150 Petra, 1611/014, 1144, 1267/400, 1267/500 (Cottongewebe mit unterschiedlichen Gewebedichten und Faserstärken), AI, AII, AIII, AIV, BI, BII, BIII, BIV, FR1, FR2, FR3, FR4, FA1, FA2, FV1a, FV1b, FV2, FV3, FV1/5, FV1/10a, FV1/10b, FA2/5a, FA2/5b, FM1, FM2, FM4, FM5, FM8 und Combat Gauze.

### 3. Darstellung der Ergebnisse und Befundbewertung

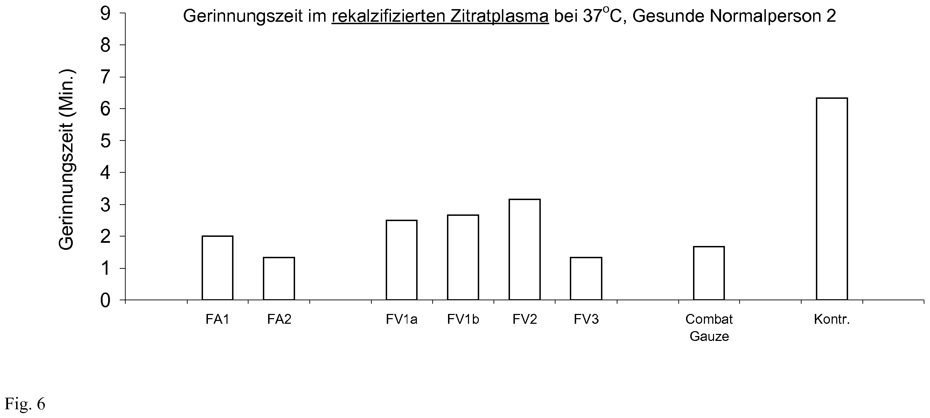

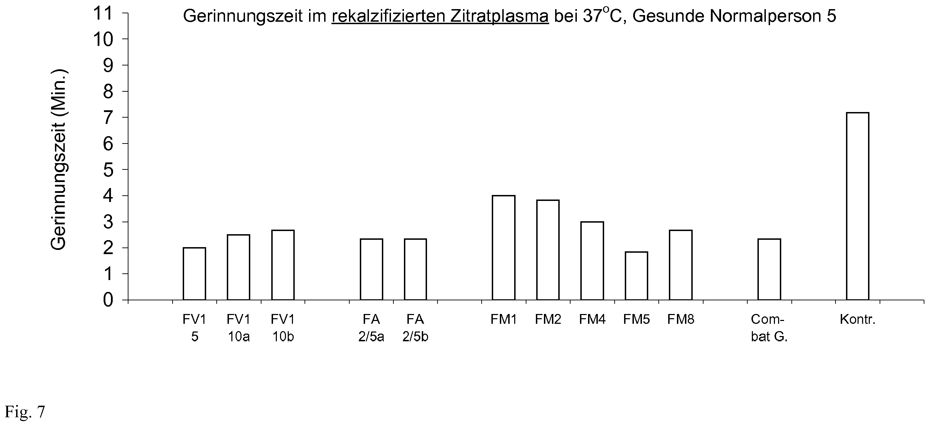

In den ersten Versuchsserien unter Verwendung der beschriebenen in vitro Testsysteme wurden die in Bezug auf die Absenkung der Gerinnungszeit im rekalzifizierten Zitratplasma und im Vollblut am besten wirkenden Pulvermineralien und Textilunterlagen ermittelt. Aus der Zusammenführung dieser Ergebnisse und deren Nutzung bei den weiteren Entwicklungsarbeiten sind die Mineral-imprägnierten Wundauflagen FA2, FV3 und FM5 hervorgegangen, die sowohl hinsichtlich der Absenkung der Gerinnungszeit, der verwendeten Textilauflage und der Stabilität der Mineralimprägnierung am besten abgeschnitten haben (Abb. 6 und 7). ABBILDUNG 6 zeigt: In vitro Testung des Einflusses Mineral-imprägnierter Baumwollunterlagen aus der FA- und FV-Reihe auf die Gerinnungszeit im rekalzifizierten Zitratplasma eines repräsentativen gesunden Blutspenders im Vergleich zum Konkurrenzprodukt Combat Gauze.

ABBILDUNG 7 zeigt: In vitro Testung des Effekts Mineral-imprägnierter Baumwollunterlagen aus der FV1-, FA2- und FM-Reihe auf die Gerinnungszeit im rekalzifizierten Zitratplasma eines repräsentativen gesunden Blutspenders im Vergleich zum Konkurrenzprodukt Combat Gauze.

Mit den optimierten Mineral-imprägnierten Baumwollmustern FA2, FV3 und FM5 wurde die Gerinnungszeit in vitro sowohl im rekalzifizierten Zitratplasma als auch im Vollblut gesunder Blutspender von 6 bis 8 auf etwas weniger als 2 Minuten verkürzt. Im Vergleich zum US-Produkt Combat Gauze senken die erfindungsgemäß bevorzugten Wundauflagen die Gerinnungszeit in Abhängigkeit vom Blutspender genauso oder etwas stärker ab. Bei den in vitro Testungen in klarem rekalzifizierten Zitratplasma fiel auf, dass das Konkurrenzprodukt Combat Gauze innerhalb weniger Sekunden zu einer deutlichen Eintrübung des Plasmas durch von der Textilunterlage abgelöstes Mineral führt. Bei der Praxisanwendung der Combat Gauze ist davon auszugehen, dass abgelöstes Mineral aus dem Wundgebiet herausgespült wird und die blutstillende Wirkung des Konkurrenzproduktes dadurch nachlässt. Bei den erfindungsgemäß bevorzugten Wundauflagen waren derartige Eintrübungen des Plasmas nicht zu beobachten, d.h. die eigenen Wundauflagen zeichnen sich gegenüber dem Konkurrenzprodukt durch eine höhere Stabilität der Mineralimprägnierung aus.

Von herausragender Bedeutung ist der mit Hilfe des etablierten in vitro Testsystems erbrachte Nachweis, dass sich die Gerinnungszeit im Vollblut und Zitratplasma von Thrombose-Risikopatienten unter der Behandlung mit Gerinnungshemmern mit den erfindungsgemäß bevorzugten erfindungsgemäßen Wundauflagen wie bei gesunden Kontrollen auf 2 Minuten absenken lässt. In Abwesenheit der Mineral-imprägnierten Wundauflagen war die Gerinnungszeit in den Kontrollansätzen bei diesen Patienten durch Einnahme von Thrombozytenaggregationshemmer und Vitamin K-Antagonisten auf 20 Min. verlängert im Vergleich zu 6 bis 8 Minuten bei gesunden Blutspendern. Dies bedeutet, dass bei Thromboserisiko-Patienten mit erhöhter Blutungsneigung unter der Einnahme von sog. "Blutverdünnem" mit den entwickelten optimierten blutstillenden Wundauflagen eine 90%ige Verringerung der Gerinnungszeit erreicht wird.

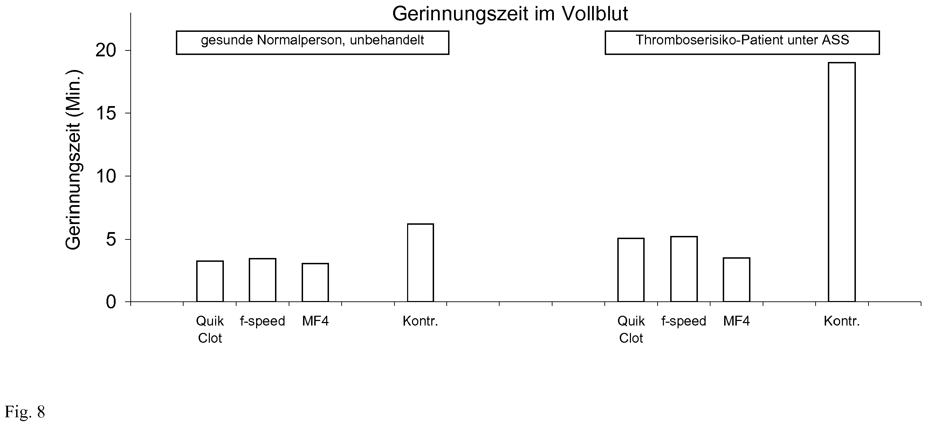

ABBILDUNG 8 zeigt: Effekte blutstillender Pulverminerale auf die Gerinnungszeit im Vollblut eines gesunden Blutspenders im Vergleich zu einem repräsentativen Thromboserisiko-Patienten, der mit dem Thrombozytenaggregationshemmer ASS behandelt wurde.

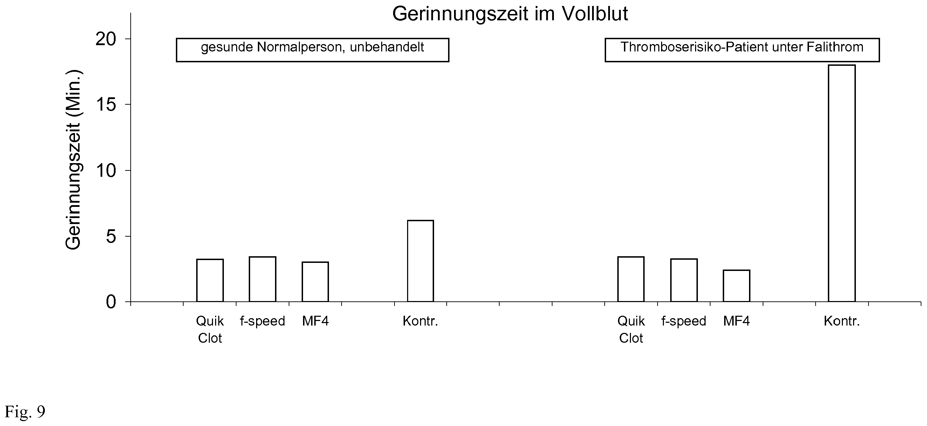

ABBILDUNG 9 zeigt: Effekte blutstillender Pulverminerale auf die Gerinnungszeit im Vollblut eines gesunden Blutspenders im Vergleich zu einem repräsentativen Thromboserisiko-Patienten, der mit dem Vitamin K-Antagonisten Falithrom behandelt wurde

### Zusammenfassung:

Die Mineral-imprägnierten erfindungsgemäßen Wundauflagen wie FA2, FV3 und FM5 sind in ihrer blutstillenden Wirkung dem Konkurrenzprodukt Combat Gauze gleichwertig, zeichnen sich aber durch eine höhere Stabilität der Mineralimprägnierung aus.

Die erfindungsgemäßen Wundauflagen sind zudem in der Lage, die durch Vitamin K-Antagonisten und Thrombozytenaggregationshemmer induzierte pharmakologische Gerinnungshemmung und die damit verbundene Blutungsneigung vollständig zu überwinden und senken die Gerinnungszeit bei den mit diesen Medikamenten behandelten Thrombose-Risikopatienten wie bei gesunden Blutspendern auf 2 Minuten ab.

### Druckverfahren bzw. Imprägnierungsverfahren des textilen Substrates

Das verwendete textile Substrat ist ein Vlies, welches im Spunlace-Verfahren hergestellt wird oder alternativ ein Nadel-Vlies.

Beim Spunlace-Verfahren werden die Fasern durch Wasserstrahl mit hohem Druck verfestigt, bei Nadel-Vliesen erfolgt die Faserverfestigung durch Nadeln.

Mittels einer Prägewalze wird auf den Vliesen eine Oberfläche mit einer Lochstruktur erzeugt, wodurch erreicht wird, dass sich die Wundauflage leichter von der versorgten Wunde wieder ablösen lässt.

Ferner kann bereits im Faserspinnprozess Halloysit-Pulver in die Milchprotein-Spinnmasse dispergiert werden, so dass der entstehende Spinnfaden bis zu 30 % Halloysit enthalten kann. Die verwendeten Vliese können unterschiedlich beladen und kombinierbare Fasern enthalten.

Das textile Substrat kann Lyocell oder andere aus Cellulose hergestellte Fasern oder aus Milch hergestellte Milchproteinfasern bzw. Mischungen aus vorgenannten, sowie Zumischungen von Halloysit und/oder Algen, die in die Fasern bereits beim Faserspinnprozess eingelagert sind, enthalten.

Zur Bedruckung mittels Siebdruckverfahren wird eine druckfähige Paste unter Verwendung von Halloysit-Pulver (250-300 g), Wasser (950 g) und Binder (50 g) hergestellt und über eine Flachdruckschablone mit Rakelgeschwindigkeit von 1,2 m/s auf das textile Substrat aufgebracht und bei ca. 120 °C anschließend getrocknet. Als wasserlöslicher Binder werden Zellulose-Gelantine-Mischungen oder Copolymere auf Styrol- und Acrylsäureesterbasis eingesetzt. Die Pastenviskosität liegt bei 60 dPas nach Haake.

Das Konfektionieren der bedruckten textilen Substrate erfolgt mittels Stoßmesser-Cutter, Laser-Cutter oder Ultraschall-Cutter um präzise Schnittränder zu erhalten. Anschließend erfolgen Sterilisierung und Verpackung.

Eine weitere beispielhafte Herstellung der Wundauflage erfolgt gemäß folgender Schritte:

### 1. Wirkmineral: natürliche Halloysite Nanotubes (HNT's)

Durch entsprechende Temperaturbehandlung in die 7 A-Form überführt und weiterhin im Niedertemperaturplasma mit bioziden Eigenschaften ausgestattet, für den verwendeten natürlichen Halloysit wird der Nachweis der Erfüllung der Reinheitsanforderungen für Kaolinum Ponderosum / Medical Kaolin geführt. Halloysite Nanotubes sind biokompatibel und unterliegen nicht der EU-Nanopartikelklassifikation (>50 % < 100 mm) und sind von der EPA als nontoxic / GRAS (4a) eingestuft.

### 2. Verwendete textile Fasern und Vliese

Bevorzugt werden folgende Fasern auch als Gemisch verwendet:
e) Lyocellfaser mit bis zu 20 % (Vol-%) Algenzusatz (in die Faser inkorporiert)
f) Gemisch aus Viskosefaser und Milchsäurefaser (50:50)
g) Milchsäurefaser mit bis zu 30 % Halloysitanteil (in die Faser inkorporiert)
h) Vlies aus 100 % Milchsäurefaser.

Die Halloysite werden durch ein Verfahren im Rahmen der Herstellung der Fasern/Spinnmasse dispergiert und dadurch in die Matrix der aus Spinnmasse entstehenden Fasern eingebunden sind.

Um das textile Substrat zu bilden, werden die Fasern nach den beispielhaften wie auch nach anderen möglichen bevorzugten Herstellungsprozessen anschließend beispielsweise zu Vliesstoff, Gewebe, Gaze, Gestrick, Gewirke oder Gelege weiter verarbeitet.

### 3. Imprägnierungstechnologie

Bevorzugt wird ein Druckverfahren (Siebdruck, Flachdruck) zum Aufbringen einer HNT-haltigen Paste auf die textile Unterlage verwendet. Dadurch ergibt sich die Möglichkeit, das Verhältnis von imprägnierter zu nicht imprägnierter Textilfläche in breiten Bereichen zu steuern, was bezüglich der Blutaufnahme und der Anhaftung an der zu behandelnden Wunde von Bedeutung ist.

Die Halloysit-Pastenherstellung erfolgt bevorzugt durch die Dispergierung von Halloysitpulver in destilliertem Wasser unter Zugabe von 1 - 5 % wasserlöslichen Bindern (basierend auf einer Zellulose-Gelantine-Komposition). Nach dem Druck entfällt ein abschließendes Absaugen, Abschwemmen oder Abrütteln des überschüssigen Mineralproduktes.

Die Anwendung zur Wundbehandlung erfolgt bevorzugt durch das direkte Aufbringen von Mineralgranulaten oder in Kombination mit textilem Mullgewebe oder Vliesen, die mit Mineralpartikeln und weiteren Additiven durch verschiedene Techniken (Tauchen, Besprühen) ganzheitlich beschichtet werden (US 2016 0193380 A1, US 907878 B2).

Wesentliche Nachteile der nach dem Stand der Technik hergestellten mineralbasierten Wundauflagen sind:
- hohe Temperaturentwicklungen bei der Anwendung (bis zu 70 ° C, was zu Verbrennungen im Wundumfeld führt) und schlechte Anwendbarkeit/Dosierung im Falle von Zeolithgranulaten und anderen Mineralgranulaten.
- Inhomogenitäten bei der Beschichtung oder Imprägnierung textiler Wundauflagen durch die bisher verwendeten Techniken und Gewebe (bis 10 pm große Mineralpartikel) und z.T. schlechte Ablösbarkeit von behandelten Wunden, sowie hohe Herstellungskosten durch den Anteil diverser Additive,
- für die bisher verwendeten natürlichen Mineralstoffe (z.B. Kaolinit, Montmorillonit) ist der Nachweis der Reinheitsanforderungen (Schwefelgehalte; Kontaminanten: Blei, Arsen, Cadmium, Quecksilber und Gehalte an anderen kristallinen Gemengeteilen), die verschiedene Parmacopeen vorgeben für im Markt befindliche Produktet nicht erbracht worden.

Die bevorzugten Wundauflagen haben diese Nachteile nicht.

Die Variabilität der Dichte des textilen Substrates (30 - 120 g/cm²) und der faserinkorporierten bzw. aufgedruckten Halloysite Nanotube-Menge (3 - 60 g/cm²), (unter 10 g/m² ist nicht erfindungsgemäß), ermöglicht eine sehr flexible Konfektionierung der textilen Wundauflagen, so dass für verschiedenste Anwendungen wie Notfallversorgung, Erste Hilfe oder chirurgische Krankenhausversorgung eine maßgerechte Varianz dargestellt werden kann.

### TIER-Versuche zur akuten Blutstillung bei stark blutenden Wunden

### 1. ZIELSTELLUNG

Das Grundanliegen der *in situ*/*vivo* Studie war es, die gerinnungs-fördernden bzw. beschleunigenden Effekte von vorher im *in vitro* Experiment erfolgreich getesteten mit Halloysiten beschichteten Gewebestücken (d.h. bevorzugte Ausführungsformen der erfindungsgemäßen Wundauflage) zu prüfen. Die potentiellen blutstillenden Wundauflagen sollten an einem stark blutenden Organ (*in situ*) getestet werden.

### 2. MATERIAL UND METHODEN

### 2.1 Studiendesign

Narkotisierte Wistar Ratten wurden mit einem Katheter für die arterielle Blutdruckmessung versehen, um den Blutdruck kontinuierlich aufzeichnen zu können. Während des Versuches wurden Flüssigkeit und Narkosenachdosierung über einen venösen Katheter verabreicht. Nach vollständiger Instrumentierung und anschließender Äquilibrierung (20 - 30 min) wurde eine Blutprobe entnommen.

In tiefer Narkose und unter Beatmung wurde entlang der Linea alba der Bauchraum geöffnet. Um einen Harn-Stau während der OP zu vermeiden, wurde in die Harnblase ein Katheter eingelegt. Die linke Niere wurde durch Abdeckung mit einem nicht saugfähigen sterilen OP Tuch und zur Seite schieben des Darmkonvolutes sichtbar und zugänglich gemacht. Der caudale Nierenpol wurde vorsichtig vom umgebenden Fettgewebe gelöst. Ein ca. 2 - 3 mm dickes Stück des caudalen Nierenpoles wurde nun entfernt. Von diesem Zeitpunkt ,0 min' an, wurde so eine massive Blutung induziert. Sofort nach Entfernung des Nierenpols wurde die zu testende Wundauflage direkt auf die Wunde gelegt.

Während der anschließenden Beobachtungszeit von 20 min wurde der Blutdruck aufgezeichnet und es erfolgte eine Volumensubstitution mittels physiologischer Kochsalzlösung + 1 % BSA (Rinderserumalbumin). Am Ende der Beobachtungszeit wurde die das im Bauchraum befindliche flüssige und geronnene Blut gesammelt und gemessen, sowie die auf der Wundauflage begutachtet.

Der mittlere arterielle Blutdruck wurde während der gesamten Beobachtungszeit aufgezeichnet. Für die Auswertung wurden innerhalb der ersten 10 Minuten nach Wundsetzung Mittelwerte über jeweils 1 min gebildet, abschließend der Mittelwert für den Zeitraum 10 - 20 min nach Wundsetzung.

### 2.2 Testgruppen

| Gruppe | Anzahl | Behandlung |
|---|---|---|
| Testgruppe 1 = AI | 8 | Wundauflage A I |
| Kontrolle | 8 | Wundauflage unbeschichtet, Basismaterial für AI und AII |
| Referenzgruppe = Combat Gauze | 8 | Wundauflage Combat Gauze (Cobat Medical Systems) |
| Testgruppe 2 = AII | 8 | Wundauflage AII |
| Testgruppe 3 = FA2(T) | 8 | Wundauflage FA2(T) |

### 2.3 Parameter

- zur Beschreibung des Blutverlustes:
   Hämatokrit (HK) vor und 20 min nach der Wundsetzung
   Menge von Blut und Gerinnsel nach 20 min Bauchraum
   Blutdruckverlauf, maximaler Blutdruckabfall, Zeit bis zum Wiedererreichen des Blutdrucks-Ausgangsniveaus
   Gewicht der Wundauflage vor und nach (+20 min) der Anwendung
- zur Beschreibung der Wundauflage:
   Beschreibung des Zustandes vor der Entfernung
   Beschreibung des Verhaltens bei der Entfernung
   Beobachtung von Nachblutungen nach Entfernung

### 3. ERGEBNISSE

### 3.1 Blutverlust

### 3.1.1 Reproduzierbarkeit der Wundgröße

Um die Reproduzierbarkeit der Wundgröße in dem gewählten Modell etwas einschätzen zu können, wurde das Gewicht des entfernten Nierenpols bestimmt und es ergaben sich für die Gruppen die in Tabelle 1 enthaltenen Mittelwerte.

**Tab. 1: Gewicht des entfernten Nierenstückes (Mittelwert ± SD)**

| | **AI** | **KO** | **Combat Gauze** | **A II** | **FA 2(T)** |
|---|---|---|---|---|---|
| Gewicht des Nierenpols [mg] | 109 ± 36 | 122 ± 44 | 140 ± 30 | 115 ± 30 | 128 ± 19 |

### 3.1.2 Blutdruck

In der Kontrollgruppe wurde der höchste Blutdruckabfall von 50 ± 25 mmHg gemessen (Abb. 10). Sowohl die Auflage des Referenzmaterials (Combat Gauze) als auch die der Testmaterialien hatte einen bis um die Hälfte reduzierten Blutdruckabfall zur Folge. Der geringste Abfall war nach Auflage von A II zu beobachten (Abb. 10). Der niedrigste Blutdruck wurde im Mittel nach 2 - 3 min gemessen.

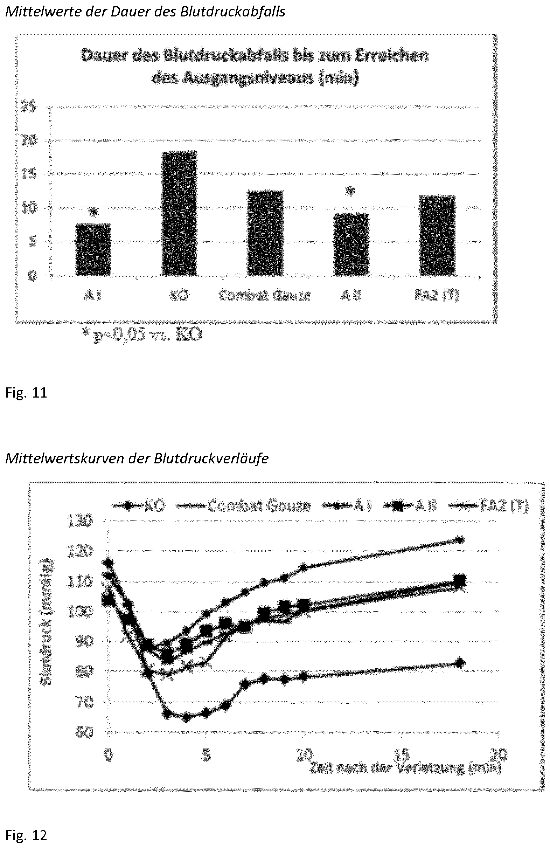

Während das Ausgangsniveau in der Kontrollgruppe im Mittel erst nach 18,8 ± 4,1 min und beim Einzeltier zum Teil gar nicht wieder erreicht wurde, stieg der Blutdruck in A I im Mittel schon nach 7,5 ± 2,1 min und in A II nach 9,1 ± 7,3 min wieder auf den Ausgangswert an (Abb. 11, Abb. 12).

### 3.1.3 Hämatokrit

Der Blutverlust hat in der Kontrollgruppe einen Abfall des Hämatokriten auf 89 % des Ausgangswertes zur Folge. Der Abfall des Hämatokriten während der Beobachtungszeit von 20 min ist in der Referenzgruppe (Combat Gauze) geringfügig (91 % des Ausgangswertes) in A I (93 %) und in A II (94 %) deutlich und in FA2(T) (95 %) signifikant geringer.

### 3.1.4 Blutmenge im Bauchraum

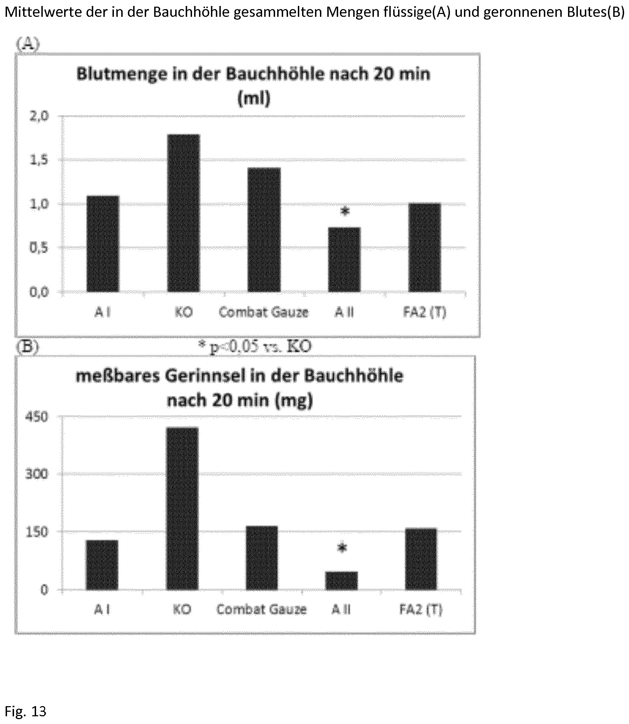

Am Ende der Beobachtungszeit hatten sich die größte Blutmenge und das größte Gerinnsel in der Kontrollgruppe angesammelt. Auf eine geringere Blutung wiesen die gemessenen Mengen in der Referenz und insbesondere in den Testgruppen hin. Am deutlichsten war der Effekt nach der Auflage von A II (Abb. 13).

### 3.1.5 Saugfähigkeit der Wundauflage

Die Bildung der Differenz von End- und Ausgangsgewicht zeigte, dass das Kontrollmaterial wenig saugfähig war, Combat Gauze, A I und A II banden mehr Blut und FA2(T) war offensichtlich am saugfähigsten und / oder gerinnungsförderndsten (Abb. 4, Abb. 13).

### 3.2. Beobachtungen zur Wundauflage

Die Wundauflage in der Kontrollgruppe erscheint optisch etwas steif und fest und wenig saugfähig. Nach 20 min Beobachtungszeit liegt sie nahezu lose am Nierenpol. Das Referenzmaterial Combat Gauze ist sehr dünn, klappt bei Blutkontakt leicht zusammen, haftet an der Wunde an und lässt sich nach 20 min leicht lösen, wobei eine geringe Nachblutung induziert wird. Die Testmaterialien A 1 und A II haften an der Niere an, lassen sich aber nach der Beobachtungszeit leicht lösen und verursachen geringe Nachblutungen. Das Testmaterial FA 2(T) (siehe oben) haftet leicht auf der Wunde an, das Blut dringt sofort durch die Auflage durch und gerinnt. Durch das Ablösen werden nur geringe Nachblutungen induziert.

### 4. Zusammenfassung

Die Beobachtung des mittleren arteriellen Blutdrucks nach dem Setzen der akuten Wunde an der Niere und der Auflage verschiedener Materialien zeigt, dass FA 2(T) vergleichbar wie das Referenzmaterial blutstillend wirkt und damit den Blutdruckabfall reduziert. Aus der noch deutlicheren Reduzierung des Blutdruckabfalls bei Wundsetzung nach Auflage von A I und insbesondere A II und auch der relativ geringen Beeinflussung des Hämatokrites lässt sich ein stärkerer blutgerinnungsfördernder Effekt der mit Halloysit beschichteten Wundauflagen ableiten. Auch die im Bauchraum gesammelte geringe Menge an Blut und Blutgerinnseln sowie die gute Fähigkeit, Blut am Material zu binden, belegen insbesondere für A II die effektivste blutgerinnungsfördernde Wirkung.

## Patentansprüche

1. Eine hämostatische Wundauflage,
**dadurch gekennzeichnet, dass**
- sie ein textiles Substrat und
- Halloysite umfasst,
die Wundauflage einen Halloysit-Anteil von 10 g/m² - 60 g/ m² umfasst.

2. Vorrichtung nach Anspruch 1,
**dadurch gekennzeichnet, dass**
das textile Substrat Verbundfasern umfasst,
wobei die Verbundfasern Mischungen umfassen, ausgewählt aus der Gruppe umfassend Viskose, Algen und/ oder Milchproteinfasern.

3. Vorrichtung nach einem der vorhergehenden Ansprüche,
**dadurch gekennzeichnet, dass**
die Verbundfasern Regeneratfasern sind.

4. Vorrichtung nach einem der vorhergehenden Ansprüche,
**dadurch gekennzeichnet, dass**
die Halloysite ausgewählt sind aus der Gruppe der Halloysit-7Å, Halloysit-10 Å und/ oder von natürlichen Mischungen daraus,
wobei insbesondere die ausgewählten Halloysite die Reinheitsanforderungen hinsichtlich von Nebenmengenanteilen und Schwermetallen erfüllen.

5. Vorrichtung nach einem der vorhergehenden Ansprüche,
**dadurch gekennzeichnet, dass**
die Halloysite Hallosite-Nanotubes mit den folgenden Abmessungen: innerer Durchmesser:10 - 20 nm (Nanometer), äußerer Durchmesser: 50 -70 nm, Länge: 0,3- 4 um (Mikrometer)
sind.

6. Vorrichtung nach einem der vorhergehenden Ansprüche,
**dadurch gekennzeichnet, dass**
die Halloysite mittels eines Bindemittels auf dem textilen Substrat aufgebracht vorliegen und das Bindemittel 90 % - 95 % deionisiertes Wasser und 5 Masse- % - 10 Masse-% Acrylat oder Gelatine umfasst.

7. Vorrichtung nach einem der vorhergehenden Ansprüche,
**dadurch gekennzeichnet, dass**
das textile Substrat eine Dichte zwischen 30 g/m² und 120 g/ m² aufweist, bevorzugt 50 g/m²
und/oder das textile Substrat ein Vlies, ein Gewebe, eine Gaze, ein Gestrick, ein Gewirke und/ oder ein Gelege ist.

8. Vorrichtung nach einem der vorhergehenden Ansprüche,
**dadurch gekennzeichnet, dass**
die Verbundfasern zwischen 3 Vol- % und 30 Vol- % Algenanteil aufweisen und/oder bis zu 100 Vol-% aus Milchproteinen bestehen.

9. Vorrichtung nach einem der vorhergehenden Ansprüche,
**dadurch gekennzeichnet, dass**
die Verbundfasern einen Halloysite-Anteil von bis zu 30 Vol-% aufweisen.

10. Vorrichtung nach einem der vorhergehenden Ansprüche,
**dadurch gekennzeichnet, dass**
die Halloysite auf dem textilen Substrat mittels Druckverfahren aufgebracht vorliegen, insbesondere mittels Siebdruck, Hochdruck, Tiefdruck und/ oder Flachdruck, bevorzugt Sieb- oder Flachdruck.

11. Vorrichtung nach einem der vorhergehenden Ansprüche,
**dadurch gekennzeichnet, dass**
die Halloysite vollflächig oder vorzugsweise 30 % - 70 % der Oberfläche des Substrates abdecken.

12. Vorrichtung nach einem der vorhergehenden Ansprüche,
**dadurch gekennzeichnet, dass**
die Halloysite bei einer Temperatur von 200 ° Celsius vorbehandelt vorliegen.

13. Vorrichtung nach einem der vorhergehenden Ansprüche, zur Verwendung bei der Blutstillung.

14. Vorrichtung nach einem der vorhergehenden Ansprüche, zur Verwendung bei der Gerinnungsförderung, insbesondere auch bei Patienten, die Blutverdünner-Medikamente einnehmen.

15. Vorrichtung nach einem der vorhergehenden Ansprüche, zur Verwendung
- als biozide Wundauflage und/oder
- bei der Wundversorgung von Menschen und/oder Tieren und/oder
- als Wundauflage für die Akutwundversorgung, insbesondere bei Schusswunden und/oder Splitterverletzungen.

## Claims

1. A hemostatic wound dressing,
**characterized in that**
- it comprises a textile substrate and
- halloysite,
wherein the wound dressing contains a halloysite content of 10 g/m² - 60 g/m².

2. The device according to claim 1,
**characterized in that**
the textile substrate comprises composite fibers,
wherein the composite fibers comprise mixtures selected from the group comprising viscose, algal and/or milk protein fibers.

3. The device according to any one of the preceding claims,
**characterized in that**
the composite fibers are regenerated fibers.

4. The device according to any one of the preceding claims, **characterized in that**
the halloysites are selected from the group of halloysite-7Å, halloysite-10Å and/or natural mixtures thereof,
wherein in particular the selected halloysites meet the purity requirements with regard to the proportions of trace components and heavy metals.

5. The device according to any one of the preceding claims,
**characterized in that**
the halloysite is halloysite nanotubes having the following dimensions: inner diameter: 10 - 20 nm (nanometers), outer diameter: 50 -70 nm, length: 0.3-4 um (microns).

6. The device according to any one of the preceding claims,
**characterized in that**
the halloysite is present on the textile substrate having been applied by means of a binder and the binder comprises 90% - 95% deionized water and 5% by mass - 10% by mass of acrylate or gelatin.

7. The device according to any one of the preceding claims, **characterized in that**
the textile substrate has a density between 30 g/m² and 120 g/m², preferably 50 g/m²
and/or the textile substrate is a nonwoven fabric, a woven fabric, a gauze, a knitted fabric, a crocheted fabric and/or a unidirectional fabric.

8. The device according to any one of the preceding claims,
**characterized in that**
the composite fibers have between 3% by volume and 30% by volume of algae and/or consist of up to 100% by volume of milk proteins.

9. The device according to any one of the preceding claims,
**characterized in that**
the composite fibers have a halloysite content of up to 30% by volume.

10. The device according to any one of the preceding claims,
**characterized in that**
the halloysite is applied to the textile substrate by means of printing processes, in particular by means of screen printing, letterpress printing, gravure printing and/or planographic printing,
preferably screen or planographic printing.

11. The device according to any one of the preceding claims,
**characterized in that**
the halloysite covers the entire surface or preferably 30% - 70% of the surface of the substrate.

12. The device according to any one of the preceding claims,
**characterized in that**
the halloysite is pretreated at a temperature of 200 °C.

13. The device according to any one of the preceding claims, for use in hemostasis.

14. The device according to any one of the preceding claims for use in promoting coagulation, in particular also in patients who are taking blood thinner medication.

15. The device according to any one of the preceding claims, for use
- as a biocidal wound dressing and/or
- in wound care for people and/or animals and/or
- as a wound dressing for acute wound care, especially for gunshot wounds and/or splinter injuries.

## Revendications

1. Pansement hémostatique,
**caractérisé en ce**
- **qu'**il comprend un substrat textile et
- des halloysites,
le pansement comprend une teneur en halloysite de 10 g/m² à 60 g/m².

2. Dispositif selon la revendication 1,
**caractérisé en ce que**
le substrat textile comprend des fibres composites,
dans lequel les fibres composites comprennent des mélanges sélectionnés dans le groupe comprenant des fibres de viscose, d'algues et/ou de protéines de lait.

3. Dispositif selon l'une des revendications précédentes,
**caractérisé en ce que**
les fibres composites sont des fibres régénérées.

4. Dispositif selon l'une des revendications précédentes,
**caractérisé en ce que**
les halloysites sont choisies dans le groupe de l'halloysite-7Â, de l'halloysite-10Å et/ou de leurs mélanges naturels,
dans lequel les halloysites choisies répondent notamment aux exigences de pureté en ce qui concerne les composants mineurs et les métaux lourds.

5. Dispositif selon l'une des revendications précédentes,
**caractérisé en ce que**
les nanotubes Halloysite Hallosite sont des dimensions suivantes : diamètre intérieur : 10 à 20 nm (nanomètres), diamètre extérieur : 50 à 70 nm,
longueur : 0,3 à 4 um (microns).

6. Dispositif selon l'une des revendications précédentes,
**caractérisé en ce que**
les halloysites sont appliquées sur le substrat textile à l'aide d'un liant et le liant comprend 90 % à 95 % d'eau désionisée et 5 % en masse à 10 % en masse d'acrylate ou de gélatine.

7. Dispositif selon l'une des revendications précédentes,
**caractérisé en ce que**
le substrat textile présente une densité comprise entre 30 g/m² et 120 g/m², de préférence de 50 g/m²
et/ou le substrat textile est un molleton, un tissu tissé, une gaze, un tricot, un tissu à maille et/ou un canevas.

8. Dispositif selon l'une des revendications précédentes,
**caractérisé en ce que**
les fibres composites présentent entre 3 % en volume et 30 % en volume d'algues et/ou sont constituées jusqu'à 100 % en volume de protéines de lait.

9. Dispositif selon l'une des revendications précédentes,
**caractérisé en ce que**
les fibres composites présentent une teneur en halloysites allant jusqu'à 30 % en volume.

10. Dispositif selon l'une des revendications précédentes,
**caractérisé en ce que**
les halloysites sont appliquées sur le substrat textile au moyen de procédés d'impression, notamment par sérigraphie, typographie, héliogravure et/ou impression planographique,
de préférence par sérigraphie ou planographie.

11. Dispositif selon l'une des revendications précédentes,
**caractérisé en ce que**
les halloysites couvrent toute la surface ou de préférence 30 % à 70 % de la surface du substrat.

12. Dispositif selon l'une des revendications précédentes,
**caractérisé en ce que**
les halloysites sont prétraitées à une température de 200° Celsius.

13. Dispositif selon l'une des revendications précédentes, destiné à être utilisé en hémostase.

14. Dispositif selon l'une des revendications précédentes, destiné à favoriser la coagulation, notamment chez les patients prenant des médicaments anticoagulants.

15. Dispositif selon l'une des revendications précédentes, pour une utilisation
- comme pansement biocide et/ou
- dans le soin des plaies des personnes et/ou des animaux et/ou
- comme pansement pour le soin des plaies aiguës, en particulier pour les plaies par balle et/ou les blessures par éclats.
